(19) Europäisches Patentamt

European Patent Office

Office européen des brevets

(11) EP 0 875 146 B1

(12) EUROPEAN PATENT SPECIFICATION

(45) Date of publication and mention
of the grant of the patent:
**31.07.2002  Bulletin 2002/31**

(51) Int Cl.[7]: **A01N 59/16**

(21) Application number: **98103781.5**

(22) Date of filing: **01.11.1994**

(54) **Anti-microbial materials**

Antimikrobielle Materialien

Produits antimicrobiens

(84) Designated Contracting States:
**AT BE CH DE DK ES FR GB GR IE IT LI LU MC NL
PT SE**
Designated Extension States:
**LT SI**

(30) Priority: **18.11.1993  US 154490
18.11.1993  US 154693
18.11.1993  US 154694
02.02.1994  US 190617**

(43) Date of publication of application:
**04.11.1998  Bulletin 1998/45**

(62) Document number(s) of the earlier application(s) in
accordance with Art. 76 EPC:
**94931472.8 / 0 729 302**

(73) Proprietor: **WESTAIM BIOMEDICAL CORP.
Fort Saskatchewan, Alberta T8L 3W4 (CA)**

(72) Inventors:
• **Burrell, Robert Edward
Sherwood Park, Alberta, T8A 4L6 (CA)**
• **Gill, Kashmir Singh
Sherwood Drive, Alberta, T8A 3W1 (CA)**
• **Morris, Larry Roy
Yarker, Ontario K0K 3N0 (CA)**
• **Apte, Prasad Shirkrishna
St. Alberta, Alberta, T8N 3W1 (CA)**
• **Precht, Roderick John
Edmonton, Alberta, T6K 3S7 (CA)**

(74) Representative:
**Hedley, Nicholas James Matthew et al
Kilburn & Strode
20 Red Lion Street
London WC1R 4PJ (GB)**

(56) References cited:
**EP-A- 0 488 269          DE-A- 2 530 487
DE-C- 819 131**

**Description**

**FIELD OF THE INVENTION**

The invention relates to methods of forming anti-microbial metal coatings, foils and powders which provide a sustained release of anti-microbial metal species when in contact with an alcohol or electrolyte.

**BACKGROUND OF THE INVENTION**

The need for an effective anti-microbial coating is well established in the medical community. Physicians and surgeons using medical devices and appliances ranging from orthopaedic pins, plates and implants through to wound dressings and urinary catheters must constantly guard against infection. An inexpensive anti-microbial coating also finds application in medical devices used in consumer healthcare and personal hygiene products as well as in biomedical/biotechnical laboratory equipment. The term "medical device", as used herein and in the claims is meant to extend to all such products.

The anti-microbial effects of metallic ions such as Ag, Au, Pt, Pd, Ir (i.e. the noble metals), Cu, Sn, Sb, Bi and Zn are known (see Morton, H.E., Pseudomonas in Disinfection, Sterilization and Preservation, ed. S.S. Block, Lea and Febiger, 1977 and Grier, N., Silver and Its Compounds in Disinfection, Sterilization and Preservation, ed. S.S. Block, Lea and Febiger, 1977). Of the metallic ions with anti-microbial properties, silver is perhaps the best known due to its unusually good bioactivity at low concentrations. This phenomena is termed oligodynamic action. In modern medical practice both inorganic and organic soluble salts of silver are used to prevent and treat microbial infections. While these compounds are effective as soluble salts, they do not provide prolonged protection due to loss through removal or complexation of the free silver ions. They must be reapplied at frequent intervals to overcome this problem. Reapplication is not always practical, especially where an in-dwelling or implanted medical device is involved.

Attempts have been make to slow the release of silver ions during treatment by creating silver containing complexes which have a lower level of solubility. For example, U.S. Patent 2,785,153 discloses colloidal silver protein for this purpose. Such compounds are usually formulated as creams. These compounds have not found wide applicability in the medical area due to their limited efficacy. The silver ion release rate is very slow. Furthermore, coatings from such compounds have been limited due to adhesion, abrasion resistance and shelf life problems.

DE-C-819131 discloses bactericidal and disinfectant agents which consist of a noble metal compound and an organic dye in solution. The noble metal is present as a complex anion. Examples given for the anions are $[AgCl_2]$, $[Ag(S_2O_3)_3]$, $[Ag(CN)_2]$, $[Ag(SCN)_2]$.

DE-A-2530487 discloses water disinfection agents which contain complex sodium-silver sulphate salts.

The use of silver metal coatings for anti-microbial purposes has been suggested. For instance, see Deitch et al., Anti-microbial Agents and Chemotherapy, Vol. 23(3), 1983, pp. 356 - 359 and Mackeen et al., Anti-microbial Agents and Chemotherapy, Vol. 31(1), 1987, pp. 93- 99. However, it is generally accepted that such coatings alone do not provide the required level of efficacy, since diffusion of silver ions from the metallic surface is negligible.

A silver metal coating is produced by Spire Corporation, U.S.A. under the trade mark SPI-ARGENT. The coating is formed by an ion-beam assisted deposition (IBAD) coating process. The infection resistant coating is stated to be non-leaching in aqueous solutions as demonstrated by zone of inhibition tests, thus enforcing the belief that silver metal surfaces do not release anti-microbial amounts of silver ions.

Given the failure of metallic silver coatings to generate the required anti-microbial efficacy, other researchers have tried novel activation processes. One technique is to use electrical activation of metallic silver implants (see Marino et al., Journal of Biological Physics, Vol. 12, 1984, pp. 93- 98). Electrical stimulation of metallic silver is not always practical, especially for mobile patients. Attempts to overcome this problem include developing in situ electrical currents through galvanic action. Metal bands or layers of different metals are deposited on a device as thin film coatings. A galvanic cell is created when two metals in contact with each other are placed in an electrically conducting fluid. One metal layer acts as an anode, which dissolves into the electrolyte. The second metal acts as a cathode to drive the electrochemical cell. For example, in the case of alternating layers of Cu and Ag, the Cu is the anode, releasing $Cu^+$ ions into the electrolyte. The more noble of the metals, Ag, acts as the cathode, which does not ionize and does not go into solution to any large extent. An exemplary device of this nature is described in U.S. Patent 4,886,505 issued Dec. 12, 1989, to Haynes et al. The patent discloses sputtered coatings of two or more different metals with a switch affixed to one of the metals such that, when the switch is closed, metal ion release is achieved.

Previous work has shown that a film composed of thin laminates of alternating, different metals such as silver and copper can be made to dissolve if the surface is first etched. In this instance, the etching process creates a highly textured surface (see M. Tanemura and F. Okuyama, J. Vac. Sci. TechnoL, 5, 1986, pp 2369-2372). However, the process of making such multilaminated films is time consuming and expensive.

Electrical activation of metallic coatings has not presented a suitable solution to the problem. It should be noted that galvanic action will occur only when an electrolyte is present and if an electrical connection between the two metals of the galvanic couple exists. Since galvanic corrosion occurs primarily at the metallic interface between the two metals, electrical contact is not sustained. Thus a continuous release of metal ions over an extended period of time is not probable. Also, galvanic action to release a metal such as silver is difficult to achieve. As indicated above, the metal ions exhibiting the greatest anti-microbial effect are the noble metals, such as Ag, Au, Pt and Pd. There are few metals more noble than these to serve as cathode materials so as to drive the release of a noble metal such as Ag at the anode.

A second approach to activating the silver metal surface is to use heat or chemicals. U.S. Patents 4,476,590 and 4,615,705, issued to Scales et al. on October 16, 1984 and October 7, 1986, respectively, disclose methods of activating silver surface coatings on endoprosthetic implants to render them bioerodible by heating at greater than 180°C or by contacting with hydrogen peroxide. Such treatments are limited in terms of the substrate/devices which can be coated and activated.

There is still a need for an efficacious, inexpensive anti-microbial material having the following properties:

- sustained release of an anti-microbial agent at therapeutically active levels;
- applicable to a wide variety of devices and materials;
- useful shelf life; and
- low mammalian toxicity.

Metal coatings are typically produced as thin films by vapour deposition techniques such as sputtering. Thin films of metals, alloys, semiconductors and ceramics are widely used in the production of electronic components. These and other end uses require the thin films to be produced as dense, crystalline structures with minimal defects. The films are often annealed after deposition to enhance grain growth and recrystallization and produce stable properties. Techniques to deposit metal films are reviewed by R.F. Bunshah et al., "Deposition Technologies for Films and Coatings", Noyes Publications, N.J., 1982 and by J.A. Thornton, "Influence of Apparatus Geometry and Deposition Conditions on the Structure and Topography of Thick Sputtered Coatings", J. Vac. Sci. Technol., 11(4), 666-670, 1974.

U.S. Patent No. 4,325,776, issued April 20, 1982 to Menzel discloses a process for producing coarse or single crystal metal films from certain metals for use in integrated circuits. The metal film is formed by depositing on a cooled substrate (below -90°C) such that the metal layer is in an amorphous phase. The metal layer is then annealed by heating the substrate up to about room temperature. The end product is stated to have large grain diameter and great homogeneity, permitting higher current densities without electromigration failures.

Silver salts such as those of nitrate, proteins, acetate, lactate and citrate have been suggested for use in anti-microbial coatings for medical devices. Silver nitrate is used in burn wound dressings in many hospitals. These salts are known to have better anti-microbial efficacy than silver metal. The mechanism by which these compounds are effective is the instant ionization/dissociation to produce the $Ag^+$ ion. The availability of the $Ag^+$ ion is reduced significantly within or in contact with bodily fluids or tissues. Due to the high chloride content of such fluids, the silver is precipitated or tied up as insoluble silver chloride (Ksp = 1.7 x $10^{-10}$M). As a consequence, excessive amounts of silver must be present within any media containing precipitants (chiefly chloride) in order to produce the same efficacy from a silver salt as would be observed in water.

Nanocrystalline materials in the forms of powders, films and flakes are materials which are single-phase or multi-phase polycrystals, the grain size of which is in the order of a few (typically <20) nanometers in at least one dimension. Fine grain powders (particle size <5 microns) may be nanocrystalline, or more typically have grain sizes >20 nm. Nanocrystalline materials and fine powders may be prepared by a number of modified gas condensation methods, wherein the material to be deposited is generated in the vapour phase, for example by evaporation or sputtering, and is transported into a relatively large volume in which the working gas atmosphere and temperature is controlled. Atoms of the material to be deposited collide with atoms of the working gas atmosphere, lose energy and are rapidly condensed from the vapour phase onto a cold substrate, such as a liquid nitrogen cooled finger. In principle, any method capable of producing very fine grain sized polycrystalline materials can be used to produce nanocrystalline materials. These methods include, for example, evaporation such as arc evaporation, electron beam vapor deposition, molecular beam epitaxy, ion beam, sputtering, magnetron sputtering and reactive sputtering (see for example, Froes, F.H. et al., "Nanocrystalline Metals for Structural Applications", JOM, 41 (1989), No. 6., pp 12 - 17; Birringer, Rainer et al., "Nanocrystalline Materials - A First Report, Proceedings of JIMIS-4; and Gleiter, H. "Materials with Ultrafine Microstructures: Retrospectives and Perspectives, NanoStructured Materials, Vol. 1, pp 1-19, 1992, and references cited therein).

## SUMMARY OF THE INVENTION

The inventors set out to develop an anti-microbial effect.

The present invention provides a method of producing an anti-microbial effect as set out in the accompanying claims.

The term "atomic disorder" as used herein includes high concentrations of: point defects in a crystal lattice, vacancies, line defects such as dislocations, interstitial atoms, amorphous regions, grain and sub grain boundaries and the like relative to its normal ordered crystalline state. Atomic disorder leads to irregularities in surface topography and inhomogeneities in the structure on a nanometre scale.

By the term "normal ordered crystalline state" as used herein is meant the crystallinity normally found in bulk metal materials, alloys or compounds formed as cast, wrought or plated metal products. Such materials contain only low concentrations of such atomic defects as vacancies, grain boundaries and dislocations.

The term "diffusion" as used herein implies diffusion of atoms and/or molecules on the surface or in the matrix of the material being formed.

The terms "metal" or "metals" as used herein are meant to include one or more metals whether in the form of substantially pure metals, alloys or compounds such as oxides, nitrides, borides, sulphides, halides or hydrides.

The term "metal powder" as used herein is meant to include metal particles of a broad particle size, ranging from nanocrystalline powders to flakes.

The term "cold working" as used herein indicates that the material has been mechanically worked such as by milling, grinding, hammering, mortar and pestle or compressing, at temperatures lower than the recrystallization temperature of the material. This ensures that atomic disorder imparted through working is retained in the material.

The term "anti-microbial effect" as used herein means that silver ions, are released into alcohol or water in concentrations sufficient to inhibit bacterial growth. The most common method of measuring anti-microbial effect is by measuring the zone of inhibition (ZOI) created when the material is placed on a bacterial lawn. A relatively small or no ZOI (ex. less than 1 mm) indicates a non-useful anti-microbial effect, while a larger ZOI (ex. greater than 5 mm) indicates a highly useful and-microbial effect. One procedure for a ZOI test is set out in the Examples which follow.

The invention extends to devices such as medical devices incorporating, carrying or coated with the anti-microbial powders or coatings. The anti-microbial coating may be directly deposited by vapour deposition onto such medical devices as catheters, sutures, implants, burn dressings and the like. An adhesion layer, such as tantalum, may be applied between the device and the anti-microbial coating. Adhesion may also be enhanced by methods known in the art, for example etching the substrate or forming a mixed interface between the substrate and the coating by simultaneous sputtering and etching. Anti-microbial powders may be incorporated into creams, polymers, ceramics, paints, or other matrices, by techniques well known in the art.

The invention provides the preparation of anti-microbial silver materials which form complex silver ions other than $Ag^+$, $Ag^{2+}$, $Ag^{3+}$, $Ag(OH)_2^-$, $Ag_2(OH)_3^-$ and $Ag_3(OH)_4^-$ in contact with an alcohol or a water based electrolyte. The complex silver ions are found to have a surprisingly greater anti-microbial efficacy than does the $Ag^+$ ion released from the silver salts of the prior art. Exemplary complex silver ions include $Ag(CN)_2^-$, $AgCN_{(aq)}$(ion pair), $Ag(NH_3)_2^+$, $AgCl_2^-$, and $Ag(S_2O_3)_2^{3-}$. Silver coatings, powders, flakes and foils prepared with atomic disorder as more fully disclosed in WO 95/13704 are exemplary of silver materials which release complex silver ions having anti-microbial efficacy. Alternatively the silver materials may be prepared as solutions, ointments, paints or suspensions containing the complex silver ions. Such silver materials have wide application, for example as coatings for medical devices, in topical anti-microbial compositions, in anti-fouling paints or coatings and as coatings for anti-microbial filters.

Thus, in accordance with a broad aspect of the invention, there is provided a method of producing an anti-microbial effect in an alcohol or a water based electrolyte as set out in the accompanying claims.

The anti-microbial form of silver material having atomic disorder has been physically characterized and has been found to have the following novel characteristics:

-    a positive rest potential, $E_{rest}$ When measured against a saturated calomel reference electrode (SCE), in 1 M potassium hydroxide;
-    preferably a ratio of temperature of recrystallization to its melting point, in degrees K, ($T_{rec}/T_m$), of less than about 0.33, and most preferably less than about 0.30;
-    preferably a temperature of recrystallization less than about 140 °C; and
-    preferably, a grain size less than about 200nm, preferably less than 140 nm and most preferably less than 90 nm.

Each of these physical characteristics, with perhaps the exception of grain size, is believed to be the result of the presence of atomic disorder in the material. The characteristics are of assistance in identifying and distinguishing such silver materials of the present invention from prior art materials or materials in their normal ordered crystalline state. The preferred novel anti-microbial silver materials have been characterized for example by XRD, XPS and SIMS analysis, as comprising substantially pure silver metal, when deposited in an inert atmosphere such as argon. However, when the working gas atmosphere contains oxygen, the materials comprise a matrix of substantially pure silver metal and one or both of, silver oxide and atoms or molecules of trapped or absorbed oxygen. A further distinguishing feature of the materials of the present invention is the presence of growth twins in the grain structure, visible from TEM analysis.

## BRIEF DESCRIPTION OF THE DRAWINGS

Figure 1 is a TEM micrograph of a sputter deposited silver coating in accordance with the invention, illustrating grain size and growth twin defects.

Figure 2 is a TEM micrograph of the film of Figure 1 after annealing, showing larger grain size and the presence of annealing twins.

## DESCRIPTION OF THE PREFERRED EMBODIMENTS

The medical devices incorporating, carrying or coated with the anti-microbial material of this invention generally come into contact with an alcohol or water based electrolyte including a body fluid (for example blood, urine or saliva) or body tissue (for example skin, muscle or bone) for any period of time such that microorganism growth on the device surface is possible. The term "alcohol or water based electrolyte" also includes alcohol or water based gels. In most cases the devices are medical devices such as catheters, implants, tracheal tubes, orthopaedic pins, insulin pumps, wound closures, drains, dressings, shunts, connectors, prosthetic devices, pacemaker leads, needles, surgical instruments, dental prostheses, ventilator tubes and the like. However, it should be understood that the invention is not limited to such devices and may extend to other devices useful in consumer healthcare, such as sterile packaging, clothing and footwear, personal hygiene products such as diapers and sanitary pads, in biomedical or biotechnical laboratory equipment, such as tables, enclosures and wall coverings, and the like. The term "medical device" as used herein and in the claims is intended to extend broadly to all such devices.

The device may be made of any suitable material, for example metals, including steel, aluminum and its alloys, latex, nylon, silicone, polyester, glass, ceramic, paper, cloth and other plastics and rubbers. For use as an in-dwelling medical device, the device will be made of a bioinert material. The device may take on any shape dictated by its utility, ranging from flat sheets to discs, rods and hollow tubes. The device may be rigid or flexible, a factor again dictated by its intended use.

Anti-Microbial Coatings

The anti-microbial coating in accordance with this invention is deposited as a thin metallic film on one or more surfaces of a medical device by vapour deposition techniques. Physical vapour techniques, which are well known in the art, all deposit the metal from the vapour, generally atom by atom, onto a substrate surface. The techniques include vacuum or arc evaporation, sputtering, magnetron sputtering and ion plating. The deposition is preferably conducted in a manner to create atomic disorder in the coating as defined hereinabove. Various conditions responsible for producing atomic disorder are useful. These conditions are generally avoided in thin film deposition techniques where the object is to create a defect free, smooth and dense film (see for example J.A. Thornton, supra). While such conditions have been investigated in the art, they have not heretofore been linked to enhanced solubility of the coatings so-produced.

The preferred conditions which are used to create atomic disorder during the deposition process include:

- a low substrate temperature, that is maintaining the surface to be coated at a temperature such that the ratio of the substrate temperature to the melting point of the metal (in degrees Kelvin) is less than about 0.5, more preferably less than about 0.35 and most preferably less than about 0.3; and optionally one or both of:

- a higher than normal working (or ambient) gas pressure, i.e. for vacuum evaporation: e-beam or arc evaporation, greater than 0.001 Pa (0.01 mT), gas scattering evaporation (pressure plating) or reactive arc evaporation, greater than 3 Pa (20 mT); for sputtering: greater than 10 Pa (75 mT); for magnetron sputtering: greater than about 1.3 Pa (10 mT); and for ion plating: greater than about 30 Pa (200 mT); and

- maintaining the angle of incidence of the coating flux on the surface to be coated at less than about 75°, and preferably less than about 30°

The coating is formed as a thin film on at least a part of the surface of the medical device. The film has a thickness no greater than that needed to provide release of metal ions on a sustainable basis over a suitable period of time. In that respect, the thickness will vary with the degree of atomic disorder in (and thus the solubility of) the coating. The thickness will be thin enough that the coating does not interfere with the dimensional tolerances or flexibility of the device for its intended utility. Typically, thicknesses of less than 1 micron have been found to provide sufficient sustained anti-microbial activity. Increased thicknesses may be used depending on the degree of metal ion release needed over a period of time. Thicknesses greater than 10 microns are more expensive to produce and normally should not be needed.

The anti-microbial effect of the coating is achieved when the device is brought into contact with an alcohol or a water based electrolyte such as, a body fluid or body tissue, thus releasing metal ions, atoms, molecules or clusters. The concentration of the metal which is needed to produce an anti-microbial effect will vary from metal to metal. Generally, anti-microbial effect is achieved in body fluids such as plasma, serum or urine at concentrations less than about 0.5 - 1.5 µg/ml.

The ability to achieve release of silver ions on a sustainable basis from a coating is dictated by a number of factors, including coating characteristics such as composition, structure, solubility and thickness, and the nature of the environment in which the device is used. As the level of atomic disorder is increased, the amount of metal ions released per unit time increases. For instance, a silver metal film deposited by magnetron sputtering at T/Tm < 0.5 and a working gas pressure of about 0.9 Pa (7 mTorr) releases approximately 1/3 of the silver ions that a film deposited under similar conditions, but at 4 Pa (30 mTorr), will release over 10 days. Films that are created with an intermediate structure (ex. lower pressure, lower angle of incidence etc.) have Ag release values intermediate to these values as determined by bioassays. This then provides a method for producing controlled release metallic coatings in accordance with this invention. Slow release coatings are prepared such that the degree of disorder is low while fast release coatings are prepared such that the degree of disorder is high.

For continuous, uniform coatings, the time required for total dissolution will be a function of film thickness and the nature of the environment to which they are exposed. The relationship in respect of thickness is approximately linear, i.e. a two fold increase in film thickness will result in about a two fold increase in longevity.

It is also possible to control the metal release from a coating by forming a thin film coating with a modulated structure. For instance, a coating deposited by magnetron sputtering such that the working gas pressure was low (ex. 2 Pa (15 mTorr)) for 50% of the deposition time and high (ex. 4 Pa (30 mTorr)) for the remaining time, has a rapid initial release of metal ions, followed by a longer period of slow release. This type of coating is extremely effective on devices such as urinary catheters for which an initial rapid release is required to achieve immediate anti-microbial concentrations followed by a lower release rate to sustain the concentration of metal ions over a period of weeks.

The substrate temperature used during vapour deposition should not be so low that annealing or recrystallization of the coating takes place as the coating warms to ambient temperatures or the temperatures at which it is to be used (ex. body temperature). This allowable ΔT, that the temperature differential between the substrate temperature during deposition and the ultimate temperature of use, will vary from metal to metal. For the most preferred metals of Ag and Au, preferred substrate temperatures of -20 to 200°C, more preferably -10°C to 100°C are used.

Atomic disorder may also be achieved, in accordance with the present invention, by preparing composite metal materials, that is materials which contain one or more anti-microbial metals in a metal matrix which includes atoms or molecules different from the anti-microbial metals.

Our technique for preparing composite material is to co- or sequentially deposit the anti-microbial metal(s) with one or more other inert, biocompatible metals selected from Ta, Ti, Nb, Zn, V, Hf, Mo, Si, Al and alloys of these metals or other metal elements, typically other transition metals. Such inert metals have a different atomic radii from that of the anti-microbial metals, which results in atomic disorder during deposition. Alloys of this kind can also serve to reduce atomic diffusion and thus stabilize the disordered structure. Thin film deposition equipment with multiple targets for the placement of each of the anti-microbial and inert metals is preferably utilized. When layers are sequentially deposited the layer(s) of the inert metal(s) should be discontinuous, for example as islands within the anti-microbial metal matrix. The final ratio of the anti-microbial metal(s) to inert metal(s) should be greater than about 0.2. The most preferable inert metals are Ti, Ta, Zn and Nb. It is also possible to form the anti-microbial coating from oxides, carbides, nitrides, sulphides, borides, halides or hydrides of one or more of the anti-microbial metals and/or one or more of the inert metals to achieve the desired atomic disorder.

Another composite material within the scope of the present invention is formed by reactively co- or sequentially depositing, by physical vapour techniques, a reacted material into the thin film of the anti-microbial metal(s). The reacted material is an oxide, nitride, carbide, boride, sulphide, hydride or halide of the anti-microbial and/or inert metal, formed in situ by injecting the appropriate reactants, or gases containing same, (ex. air, oxygen, water, nitrogen, hydrogen, boron, sulphur, halogens) into the deposition chamber. Atoms or molecules of these gases may also become absorbed or trapped in the metal film to create atomic disorder. The reactant may be continuously supplied during deposition for codeposition or it may be pulsed to provide for sequential deposition. The final ratio of anti-microbial metal(s) to reaction product should be greater than about 0.2. Air, oxygen, nitrogen and hydrogen are particularly preferred reactants.

The above deposition techniques to prepare composite coatings may be used with or without the conditions of lower substrate temperatures, high working gas pressures and low angles of incidence previously discussed. One or more of these conditions is preferred to retain and enhance the amount of atomic disorder created in the coating.

It may be advantageous, prior to depositing an anti-microbial in accordance with the present invention, to provide an adhesion layer on the device to be coated, as is known in the art. For instance, for a latex device, a layer of Ti, Ta or Nb may be first deposited to enhance adhesion of the subsequently deposited anti-microbial coating.

Anti-Microbial Powders

Anti-microbial powders, including nanocrystalline powders and powders made from rapidly solidified flakes or foils, can be formed with atomic disorder so as to enhance solubility. The powders either as pure metals, metal alloys or compounds such as metal oxides or metal salts, can be mechanically worked or compressed to impart atomic disorder. This mechanically imparted disorder is conducted under conditions of low temperature (i.e. temperatures less than the temperature of recrystallization of the material) to ensure that annealing or recrystallization does not take place. The temperature varies between metals and increases with alloy or impurity content.

Anti-microbial powders produced in accordance with this invention may be used in a variety of forms, for instance in topical creams, paints or adherent coatings. Alternatively, the powder may be incorporated into a polymeric, ceramic or metallic matrix to be used as a material for medical devices or coatings therefor.

Fine Grain or Nanocrystalline Materials of Anti-Microbial Metals

Methods of forming fine grain or nanocrystalline materials from the vapour phase are well known and documented in the literature. For instance, nanocrystalline materials may be formed by a modified standard inert-gas condensation technique. The material to be deposited is evaporated from an electrically heated boat or crucible into an inert gas atmosphere such as argon or helium with a pressure of about 5 to 7 Torr. The temperature of the boat has to be high enough to obtain a substantial vapour pressure of the material of interest. For metals, a temperature about $100°C$ above the melting point of the metal will typically provide an adequate vapour pressure. Due to interatomic collisions with the working gas atmosphere atoms, the evaporated atoms of the material lose their kinetic energy and condense onto a cold finger or substrate held at about 77 K (liquid nitrogen cooled) in the form of a lose powder or friable flakes or film, the grain size of which is less than about 20 nm. With respect to powders or flakes, a high vacuum (less than $5 \times 10^{-6}$ Pa) is restored and the powder or flakes are stripped off from the cold finger and collected in a cold trap.

Fine grain materials are produced analogously in gas condensation/vapour deposition processes, as is known in the art. This is typically achieved by altering the cold finger or substrate temperature and the gas pressure to allow the particle to coarsen to the desired size which is preferably under 5000 nm.

Fine powders/nanocrystalline powders of anti-microbial metals prepared in accordance with the known prior an processes have been tested and found not to have sufficient anti-microbial efficacy. In order to introduce atomic disorder into the materials at a level which is sufficient to produce an anti-microbial effect, the anti-microbial metal, alloy or compound to be deposited is co-, sequentially or reactively deposited in a matrix with atoms or molecules of a different material (dopant) under conditions such that atomic disorder is created and retained in the matrix. The different material is selected from inert biocompatible metals, such as Ta, Ti, Nb, B, Hf, Zn, Mo, Si and Al, most preferably Ta, Ti and Nb. Alternatively the different material is an oxide, nitride, carbide, boride, sulphide or halide of either or both of an anti-microbial metal or of the biocompatible metal. A further alternative is to introduce the different material from the working gas atmosphere, either by reactive deposition or by absorbing or trapping atoms or molecules from the working gas into the matrix. Working gas atmospheres containing oxygen, nitrogen, hydrogen, boron, sulphur and halogens may be used. Working gas atmospheres including oxygen are most preferred, such that the matrix of anti-microbial metal includes either or both of trapped oxygen and oxides of the anti-microbial metal.

A further technique for forming anti-microbial powders of the present invention is to form coatings containing atomic disorder in the manner set out above onto an inert, preferably biocompatible, particulate material such as talc, bentonite, cornstarch or ceramics such as alumina. The particles may be coated by physical vapour deposition techniques under conditions to create atomic disorder, as set forth above in respect of the anti-microbial metal coatings. Alternatively, the powders can be coated by adapting a vapour deposition process, for instance by passing a vapour of the anti-microbial material through a fixed porous bed of the powders, by fluidizing the powder bed in the anti-microbial metal vapour phase, or by letting the powder fall through a vapour of the anti-microbial material. In all cases, the powder could be cooled and/or the working gas atmosphere could be altered to include a different material (ex. oxygen), in order to produce the desired degree of atomic disorder.

Activation of Anti-Microbial Materials

Irradiation of anti-microbial materials (powders, nanocrystalline powders, foils, coatings or composite coatings of anti-microbial metals) which contain atomic disorder formed by any of the above-described procedures, will further activate or enhance the anti-microbial effect. Thus, even materials having a low level of atomic disorder may be activated to an anti-microbial level.

Irradiation is performed with any low linear energy transfer form of radiation, including beta, gamma and x-rays. Gamma radiation at a dose of 1 Mrad or greater is preferred. Since gamma radiation is an acceptable method of sterilization of medical devices, activation and sterilization may be achieved simultaneously through the irradiation process of the present invention.

The irradiation step is preferably conducted such that the anti-microbial material being irradiated is oriented generally perpendicular to the incoming radiation (rather than parallel). A further enhancement of the and-microbial effect can be achieved by conducting the irradiation step with a dielectric material adjacent to, or preferably sandwiched around the anti-microbial material. Exemplary dielectrics include oxides of Si, Ti, Ta and Al. Silicon oxide surfaces are preferred. It is believed that the dielectric material provides forward scattering of electrons into the anti-microbial coating.

Without being bound by the same it is believed that the irradiation step is causing one or more of the following changes in the anti-microbial material:

1) creating further atomic disorder, such as point defects;
2) enhancing oxygen adsorption/chemisorption to the surface of the anti-microbial material;
3) activating trapped dopant atoms or molecules such as oxygen to $O^+$ or $O_2^-$; and
4) creating broken or dangling bonds at the surface.

With respect to the second and third proposed mechanisms, it is possible that oxygen adsorption/chemisorption and/or activation creates a super saturated concentration of $O_2$, $O^+$ or $O_2^-$ species in or on the anti-microbial metal surface, which results in the more rapid dissolution of the anti-microbial metal or species thereof into an aqueous environment through the generation of various chemical species of the anti-microbial metal, including oxides and hydroxides.

Silver Materials Forming Complex Silver Ions

In accordance with the invention, silver materials are prepared which form complex silver ions other than $Ag^+$, $Ag^{2+}$ $Ag^{3+}$, $Ag(OH)_2^-$, $Ag_2(OH)_3^-$ and $Ag_3(OH)_4^-$ when the material is contacted with an alcohol or a water based electrolyte. Exemplary complex silver ions shown to demonstrate an anti-microbial effect include $Ag(CN)_2^-$, $AgCN_{(aq)}$ (ion pair), $Ag(NH_3)_2^+$, $AgCl_2^-$, and $Ag(S_2O_3)_2^{3-}$. These silver materials forming complex silver ions have wide application, for instance, as anti-microbial coatings for medical devices, as anti-microbial powders for medical or pharmaceutical use, as anti-fouling paints, coatings or compositions, anti-microbial coatings for filters and the like.

It should be understood that the phrase "silver materials which form complex silver ions other than $Ag^+$, $Ag^{2+}$ $Ag^{3+}$ $Ag(OH)_2^-$, $Ag_2(OH)_3^-$ and $Ag_3(OH)_4^-$" as used herein and in the claims is not intended to exclude silver materials which form one or more of such silver ions in addition to other complex silver ions when the material contacts an alcohol or a water based electrolyte. The notation $Ag^+$, $Ag^{2+}$ $Ag^{3+}$ $Ag(OH)_2^-$, $Ag_2(OH)_3^-$ and $Ag_3(OH)_4^-$ refers to these ions in solution and includes solvated forms. The term complex silver ions as used herein and in the claims is not intended to include silver ions stabilized with strong oxidizing agents, such as persulphate and periodate, to prevent the reduction of the silver ions.

The anti-microbial coatings, powders and foils of the present invention, when created with atomic disorder as above described, are exemplary of silver materials which form complex silver ions other than $Ag^+$ so as to cause an anti-microbial effect It is believed that the complex silver ions which may be formed when such silver materials contact an alcohol or water based electrolyte, are one or more of the negative ions $Ag(OH)_2^-$, $Ag_2(OH)_3^-$ and $Ag_3(OH)_4^-$.

Silver materials which form complex silver ions may also be prepared by bringing a silver metal, compound or salt into an environment containing excessive amounts of a cationic, anionic or neutral species with which it is desired to complex silver. For example, the negative complex silver ion $AgCl_2^-$ can be generated by placing a silver salt such as $AgNO_3$ in an aqueous medium with an elevated concentration of the $Cl^-$ ion. $AgNO_3$/NaCl or AgCl/NaCl mixtures, solutions or suspensions can form the $AgCl_2^-$ ion. This $AgCl_2^-$ ion may also be generated with mixtures of silver powder with NaCl. Preferably the silver powder is one which is prepared in accordance with the present invention so as to contain atomic disorder, but bulk silver may also be activated in this manner. Bulk silver powder, fine grain (<140 nm) and nanocrystalline (<20 nm) powders may be used. Similarly, the ion $Ag(NH_3)_2^+$ can be formed in aqueous solution by adding silver salts to excess ammonium hydroxide. The ion $Ag(S_2O_3)_2^{3-}$ may be formed in aqueous solution by adding silver salts to excess sodium thiosulphate. The ion $Ag(CN)_2^-$ may be formed in aqueous solution by adding excess potassium cyanide to silver cyanide.

The silver materials forming complex silver ions may be formulated for use in many forms, including for example, powders, suspensions, solutions, ointments or coatings. For instance, a pharmaceutical composition to generate the $AgCl_2^-$ ion can be formulated as a mixture of the salts $AgNO_3$/NaCl or as a mixture of NaCl with a silver powder, preferably one containing atomic disorder. These mixtures of the silver material might be pre-formulated as a solution, suspension or ointment with a sterile aqueous or saline solution and pharmaceutically acceptable carriers, diluents, exipients and the like. Alternatively the silver material might be provided as the mixtures of silver powder/

NaCl salt or AgNO$_3$/NaCl, for later formulation by the end user.

Physical/Chemical Characteristics of Anti-Microbial Silver Material

The modified metal materials formed in accordance with the present invention so as to contain atomic disorder which leads to enhanced release of the metal species have novel physical characteristics when compared with materials in their normal ordered crystalline state. Silver materials made in accordance with the present invention have been characterized as having the following novel characteristics:

- a positive rest potential, E$_{rest}$ for example, when measured against an SCE reference electrode in a 1 M KOH solution;
- preferably a ratio of temperature of recrystallization to melting temperature less than 0.33, and most preferably less than 0.30;
- preferably a temperature of recrystallization less than about 140 °C; and
- preferably a grain size less than about 200nm, more preferably less than 140nm and most preferably less than 90nm.

Analysis of the silver materials by XRD, XPS and SIMS techniques confirms the chemical nature and content of the film as silver metal, and in the event that the material is formed with oxygen in the working gas atmosphere, one or both of silver oxide and trapped oxygen. TEM analysis reveals growth twins in the silver material, which are converted to annealed twins when the materials are annealed above the temperature of recrystallization.

The invention is further illustrated by the following non-limiting examples.

Example 1

A medical suture material size 2/0, polyester braid was coated by magnetron sputtering 20.3 cm diameter (8 in.) from planar silver and copper magnetron cathodes to form an Ag-Cu-alloy on the surface to a thickness of 0.45 microns, using either argon gas working pressures of 0.9 Pa (7 mTorr) or 4 Pa (30 mT) at 0.5 KW power and a T/Tm ratio of less than 0.5. The total mass flow of gas was 700 sccm (standard cubic centimeters per minute).

The anti-microbial effect of the coatings was tested by a zone of inhibition test. Basal medium Eagle (BME) with Earle's salts and L-glutamine was modified with calf/serum (10%) and 1.5 % agar prior to being dispensed (15 ml) into Petri dishes. The agar containing Petri plates were allowed to surface dry prior to being inoculated with a lawn of *Staphylococcus aureus* ATCC# 25923. The inoculant was prepared from Bactrol Discs (Difco, M.) which were reconstituted as per the manufacturer's directions. Immediately after inoculation, the materials or coatings to be tested were placed on the surface of the agar. The dishes were incubated for 24 h at 37°C. After this incubation period, the zone of inhibition was measured and a corrected zone of inhibition was calculated (corrected zone of inhibition = zone of inhibition - diameter of the test material in contact with the agar).

The results showed no zone of inhibition on the uncoated suture, a zone of less than 0.5 mm around the suture coated at 0.9 Pa (7 mTorr) and a zone of 13 mm around the suture coated at 4 Pa (30 mTorr). Clearly the suture coated in accordance with the present invention exhibits a much more pronounced and effective anti-microbial effect.

Further examples of the beneficial anti- microbial effect of atomic disorder are given in EP-0 729 302. EP-0 729 302 also contains irritation and sensitivity tests performed on animals.

Example 2

This example is included to demonstrate the generation of silver complex ions which are distinct from the Ag$^+$ ion and which are highly efficacious in generating an anti-microbial effect. The example provides comparative diffusion and zone of inhibition (ZOI) data for various silver solutions.

Solutions were prepared to generate 10,000 ppm Ag as AgNO$_3$, Ag(NH$_3$)$_2$$^+$, Ag(CN)$_2^-$, Ag(S$_2$O$_3$)$_2$$^{3-}$ and Ag (protein).

The silver solutions were prepared as follows:

1) Ag(S$_2$O$_3$)$^{3-}$ - 2.66 g of AgCl were dissolved in 150 ml of deionized water. 17.22 g of Na$_2$(S$_2$O$_3$) were added and the volume was brought up to 200 ml with deionized water.

2) Ag(CN)$_2^-$ - Equal volumes of 12.5 g/L AgCN and 50g/L KCN were mixed.

3) Ag(protein) - Two silver protein samples were tested. Silver protein powder (0.5 g of Sigma S-6767, lot # 121H3437, 20% Ag) were added to 10 ml of deionized water. Silver protein powder (1.25 g of Sigma S-9017, lot # 33H3456, 8% Ag) were added to 10 ml of deionized water.

4) $Ag(NH_3)_2^+$ - Silver nitrate was added to ammonium hydroxide to form a black precipitate. To this solution was added dropwise additional ammonium hydroxide until the precipitate redissolved, leaving the complex silver ion $Ag(NH_3)_2^+$ in solution.

Also prepared were control solutions containing the same concentrations of nitrate, ammonia, cyanide and thiosulphate as was present in the test solutions. The anti-microbial effect of the test solutions was tested by a zone of inhibition test. A sensi disc (cellulose, 6mm diameter) containing 25 microlitres of each of the test solutions was placed in the middle of a MHA (Difco media) plate. The silver complexes or ions in the sensi disc were allowed to diffuse for 4 hours on the MHA plate stored in a 37°C incubator. After 4 hours, the sensi disc was removed from the plate and analyzed for silver content using neutron activation analysis (NAA, University of Alberta Slowpoke Reactor Facility). A further set of plates were used to measure zones of inhibition against *S. aureus* for each of the silver complexes or ions in the sensi discs. Samples of the agar were taken from the plates from two locations - the edge of the zone of inhibition and underneath the discs. The agar samples were analyzed for silver content by NAA. The control solutions were tested for anti-microbial effect and were found to cause no zone of inhibition. The results are set forth in Table 1.

Table 1

| Anti-Microbial Effect of Ag+ Ion Compared to Silver Complex Ions | | | | |
|---|---|---|---|---|
| Test Solution | ZOI (mm) | Silver Content (ppm) | | Edge of ZOI |
| | | In Disc | Under Disc | |
| $Ag(NO)_3$ | 6 | 9000 | 100 | 1.8 |
| $Ag(NH_3)_2^+$ | 18 | 7300 | 221 | 1.7 |
| $Ag(CN)_2^-$ | 70 | 1400 | 420 | 4.3 |
| $Ag(S_2O_3)_2^{3-}$ | 36 | * | * | * |
| $Ag(protein)$ | 6 | * | * | * |

∗ Not measured

The above results indicate that silver salts or compounds known to dissociate to produce the $Ag^+$ ion (ex. silver nitrate and silver proteins) have a limited anti-microbial effect (6mm ZOI). The anti-microbial effect is greater for silver compositions which release silver complex ions other than $Ag^+$ (ex. $Ag(NH_3)_2^+$, $Ag(CN)_2^-$ and $Ag(S_2O_3)_2^{3-}$). It is also apparent that the silver complex ions are able to diffuse further in the agar medium than the $Ag^+$ ion, thereby achieving an anti-microbial effect further from the silver source.

Without being bound by the same, it is believed that the $Ag^+$ ion is less efficacious in its anti-microbial effect because it readily precipitates in the agar medium with chloride ions known to be present The silver complex ions on the other hand demonstrate a higher level of anti-microbial effect and more rapid diffusion. The silver complex ions are also believed not to precipitate with chloride ions to such an extent, making them more suitable for use in industrial systems or with medical devices and the like which come into contact with fluids containing chloride ions.

Example 3

This example provides comparative diffusion data and zone of inhibition data for several silver anti-microbial coatings.

Three silver films were sputtered under the conditions set forth in Table 2.

TABLE 2.

| Sputterine Conditions | Film 1 | Film 2 | Film 3 |
|---|---|---|---|
| Target (20.3 cm dia) | 99.99% Ag | 99.99% Ag | 99.99% Ag |
| Working Gas | 99/1 wt% Ar/$O_2$ | 99/1 wt% Ar/$O_2$ | 99/1 wt% |
| Working Gas Pressure | 0.7 Pa | 5.3 Pa | 5.3 Pa |
| Total Mass Flow | 700 sccm | 700 sccm | 700 sccm |
| Power | 0.5 kW | 0.5 kW | 0.05 kW |
| Substrate Temperature | 21°C | 21°C | 21°C |
| Anode/Cathode Distance | 100 mm | 100 mm | 100 mm |

The coatings were tested for anti-microbial activity by a ZOI test, as set forth in previous examples. Silver content was measured by NAA after 4 hours diffusion in the agar medium, as set forth in Example 2. The comparative results are set out in Table 3.

Table 3

| Anti-Microbial Effect of Silver Coatings | | | | |
|---|---|---|---|---|
| Test Film | Ag Species | CZOI (mm) | Silver Content (ppm) | |
| | | | Under Film | Edge of ZOI |
| Film 1 | $Ag^+$ | 2 | 35 | 0.8 |
| Film 2 | AgX[1] | 12 | 8.5 | 0.7 |
| Film 3 | $Ag^+$ + AgX[1] | 12 | 654 | 0.4 |

[1] AgX is a silver complex ion or ion pair.

For Film 1, which releases predominantly $Ag^+$ ions, a small ZOI is produced, with the silver being precipitated as AgCl below the film. For Film 2, a much larger ZOI (6X) is produced with ¼ the amount of silver being precipitated under the wafer. This suggests that a silver complex ion different than $Ag^+$ is formed which diffuses more readily. It is believed that the diffusion is accelerated as a result of the nature of the complex silver species. Film 3 releases much more silver than Films 1 or 2, but the bulk of the silver is in the form of $Ag^+$ which precipitates as AgCl under the film. However, the size of the ZOI indicates that, in addition to $Ag^+$, a complex silver ion with much greater mobility than $Ag^+$ is generated. It is believed that one or more of the negative silver hydroxyl ions $Ag(OH)_2^-$, $Ag_2(OH)_3^-$, or $Ag_3(OH)_4^-$ are generated. In that chloride is in the agar medium, negative silver hydroxyl-chloro complexes may form.

Example 4

This example is included to demonstrate the preparation of complex ions of silver cyanide, and the anti-microbial effect of such ions.

A silver cyanide bath typically used in electroplating was tested for anti-microbial effect using 25 microlitres of bath on a sensi disc in a standard ZOI test The silver cyanide bath contained 37 g/L silver cyanide, 45 g/L potassium cyanide and 30 g/L potassium carbonate. The resulting ZOI covered the entire plate, indicating a corrected ZOI greater than 94 mm. The maximum amount of silver that was available in the AgCN bath was 30,000 ppm. From previous work it is known that this concentration as $AgNO_3$ would not yield a ZOI greater than 6 mm. The effect of the cyanide ion alone was determined by placing 25 microlitres of 45 g/L KCN on a sensi disc and repeating the ZOI test. A corrected ZOI of 12.5 mm was produced. A solution of AgCN in distilled water (37 g/L) was similarly tested for a ZOL A corrected ZOI of 14 mm was observed.

The molar ratio of silver ion to cyanide ion in the bath was 0.37:1. This favours the formation of a negative silver cyanide complex $Ag(CN)_2^-$ or AgCN(aq) as an ion pair. The above results demonstrate that these complex silver ions have anti-microbial efficacy and increased mobility within an agar medium.

Thin strips of filter paper were treated with 50 microlitres of either a silver nitrate solution (10,000 ppm Ag) or a potassium cyanide solution (6,400 ppm $CN^-$). The strips were subjected to a standard ZOI test on the MHA plate. Silver nitrate control strips gave a corrected ZOI of 8 mm, while the KCN control strips gave no ZOL When one of each of the silver nitrate and potassium cyanide strips were placed on the MHA plate at right angles to each other, the corrected ZOI was 30 mm from the silver nitrate strip and 22 mm from the potassium cyanide strip.

This result demonstrates that a complex silver ion resulting from the combination of silver nitrate and potassium cyanide in the media has greater anti-microbial efficacy than either solution alone.

Example 5

This example is included to demonstrate the anti-microbial efficacy of a complex silver ion of silver chloride.

Silver chloride was pressed into a 0.2 g pellet at 413,550 kPa (60,000 psi) and tested using a standard ZOI test on MHA plates. An 8 mm zone resulted. A mixture of 0.15g AgCl and 0.05 g NaCl was pressed into a pellet at 60,000 psi and similarly tested. A 24 mm zone was observed.

The increased concentration of the available chloride ion favours the formation of the complex silver ion $AgCl_2^-$, which is demonstrated above to have improved anti-microbial efficacy over AgCL

A silver nitrate solution (10,000 ppm Ag) was tested with sensi discs (25 microlitres) in a ZOI test. A 6 mm zone was observed. The same concentration of $AgNO_3$ was tested on an agar plate which had been supplemented with 5% NaCl. A 20 mm zone was observed, indicating improved anti-microbial efficacy. A control plate of agar supplemented with 5% NaCl did not inhibit bacterial growth (*S. aureus*).

It is believed that the higher concentrations of the chloride ion favoured the formation of the complex silver ion $Ag(Cl)_2^-$. This species shows three times the anti-microbial efficacy of $Ag^+$ from silver nitrate.

Example 6

This example illustrates the structural and chemical characteristics of sputter deposited silver films that exhibit good anti-microbial activity (corrected zone of inhibition, CZOI) using the zone of inhibition test as set forth in previous examples. The films were produced by sputtering of a solid 20.3 cm dia planar silver magnetron target onto silicon wafer substrates (100 mm from the target) under the conditions summarized in Table 4. The total mass gas flow was 700 sccm. The ratio of substrate temperature to melting point of silver (1234K), $T/T_m$, was less than 0.3, the thickness of the film was nominally 3000Å and the angle of incidence in each case was 90° (normal incidence). The characteristics of as deposited silver as well as those that were subsequently annealed (in air at 140°C for 90 minutes) are described in this example. The films were characterized in terms of structural (grain size, type of defects, recrystallization) and chemical properties (dopant concentration (wherein dopant refers to atomic %O or oxide content), and electrochemical rest potential). The results are summarized in Tables 5 and 6.

The dopant concentration in the film was measured using x-ray photoelectron spectroscopy (XPS) and secondary ion mass spectrometry (SIMS). In the XPS technique a monochromatized Al K$\alpha$ x-ray beam was used as the incident beam. A 4kV Ar ion beam was rastered over a 2 mm x 2 mm area in order to remove surface contaminants and expose a fresh surface for XPS analysis. A positive cesium ion beam at 12.5 kV was employed for the SIMS analysis. The dopant concentration computed from XPS and SIMS data is summarized in Tables 15 and 16 for both as deposited and annealed films. It can be seen that one preferred characteristic of biologically active silver films in accordance with the invention is the presence of a dopant. The XPS and SIMS data further showed that the dopant, which in the present case was oxygen or both silver oxide and oxygen, was not chemically bound to the silver atoms in the bulk film. Moreover, the dopant as oxygen was incorporated in such amounts as to exceed the room temperature solid solubility in silver.

The grain size of as deposited and annealed films was measured from images taken with a transmission electron microscope (TEM). These data, reported in Tables 10 and 11, demonstrate that anti-microbial active silver films of this invention have an average grain size smaller than 200 nm. Active films, as deposited, had an average grain size less than about 140 nm. The most active films, as deposited, had an average grain size less than 90 nm. In addition, high resolution transmission electron microscopy showed that the onset of recrystallization (Trec) commenced at about 90°C. Grain growth of these fine grained, biologically active films, occurred at temperatures well below 0.33 $T_m$, where $T_m$ is the melting point of silver in degrees K, in particular below 140°C. In general, recrystallization diminished anti-microbial activity. However, coatings with higher levels of silver oxide (coatings 3 and 6) retained anti-microbial activity after annealing. It is believed that the oxide pins sufficient atomic defects so as to retain anti-microbial activity after annealing.

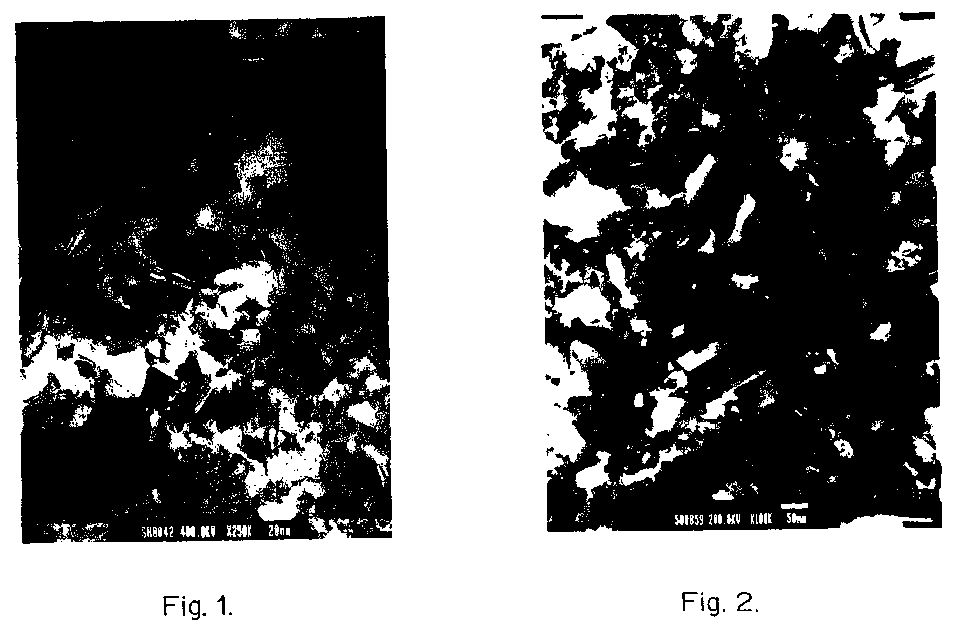

The TEM analysis further indicated that biologically active silver films contained a number of growth twins. Upon annealing in air at 140°C for 90 minutes these growth twins disappeared and annealing twins appeared. These latter twins were, however, the result of recovery, recrystallization and grain growth which transformed the silver film into a lower energy state. Evidently, these deposited silver films, along with the associated growth twins that underwent such grain growth, were in a higher energy state. Thus, the presence of these aforementioned defects in the as deposited films is a distinguishing characteristic of anti-microbial coatings in accordance with this invention. Figures 1 and 2 are TEM micrographs showing the grain sizes and twins observed in as deposited and annealed silver films respectively.

The rest potential of the silver films was measured in one molar (1M) potassium hydroxide (KOH) solution using a saturated calomel electrode (SCE) as the reference electrode. Tables 5 and 6 show that the silver films exhibited anti-microbial behaviour only when the rest potential was positive. No biological activity was observed when the rest potential was negative.

Table 4

| Growth Conditions for Sputter Deposited Silver Anti-microbial Coatings | | |
|---|---|---|
| ID Number | GROWTH CONDITIONS | | |
| | Gas Composition | Pressure Pa (mTorr) | Power(kW) |
| 1 | 99% Ar, 1% O | 1.3 (10) | 0.10 |

Table 4   (continued)

| Growth Conditions for Sputter Deposited Silver Anti-microbial Coatings | | | |
|---|---|---|---|
| ID Number | GROWTH CONDITIONS | | |
| | Gas Composition | Pressure Pa (mTorr) | Power(kW) |
| 2 | 99% Ar, 1% O | 1.3 (10) | 0.50 |
| 3 | 99% Ar, 1% O | 5.3 (40) | 0.05 |
| 4 | 99% Ar, 1% O | 5.3 (40) | 0.10 |
| 5 | 99% Ar, 1% O | 5.3 (40) | 0.50 |
| 6 | 80% Ar, 20% O | 5.3 (40) | 0.10 |

Table 5

| Structural Characteristics of Sputter Deposited Silver Anti-microbial Coatings | | | | | |
|---|---|---|---|---|---|
| Growth Condition ID Number | As Deposited | | | | |
| | Grain Size (nm) | Dopant Concentration Atomic %O | Rest Potential mV (vs SCE)[1] | Defects | C Z O I (mm) |
| 1 | 37 | 5.5 | +125 | Growth twins | 9 |
| 2 | 148 | 0 | -342 | - | 2 |
| 3 | 21 | 20.0* | +150 | Growth twins | 10 |
| 4 | 19 | 8.0 | +135 | Growth twins | 7 |
| 5 | 41 | 3.4 | +131 | Growth twins | 9 |
| 6 | 22 | 58.0* | +146 | - | 8 |
| Bulk Silver | >200 | 0 | -170 | - | <1 |

* as $Ag_2O$
[1] These values are subject to variability of $\pm20$ mV
- not measured

Table 6

| Structural Characteristics of Annealed Silver Anti-microbial Coatings | | | | | |
|---|---|---|---|---|---|
| Growth Condition ID Number | Annealed at 140°C. 90 Minutes | | | | |
| | Grain Size (nm) | Dopant Concentration atomic %O | Rest Potential mV (vs SCE)[1] | Defects | CZQI (mm) |
| 1 | 91 | - | -6 | Annealing twins | 1 |
| 2 | 135 | 0 | -224 | Annealing twins | 0 |
| 3 | 130 | 16.0* | +121 | Annealing twins | 10 |
| 4 | 73 | 0.8 | +33 | Annealing twins | 8 |
| 5 | 132 | 0.7 | -29 | Annealing twins | 0 |
| 6 | - | 31.0* | +127 | - | 8 |
| Bulk Silver | >200 | 0 | -170 | - | <1 |

* as $Ag_2O$
[1] These values are subject to variability of $\pm20$ mV
- not measured

All publications mentioned in this specification are indicative of the level of skill of those skilled in the art to which this invention pertains.

## Claims

1. A method of producing an anti-microbial effect in an alcohol or water based electrolyte, comprising;

   (a) preparing a silver material such that it forms complex silver ions other than $Ag^+$, $Ag^{2+}$, $Ag^{3+}$, $Ag(OH)_2^-$, $Ag_2(OH)_3^-$ or $Ag_3(OH)_4^-$, in an amount so as to produce an anti-microbial effect in contact with an alcohol or a water-based electrolyte that is greater than that produced by an equivalent amount of silver as $Ag^+$, wherein the silver material is either in the form of a fine grain or nanocrystalline powder, optionally for use in the preparation of a topical anti-microbial composition, or in the form of an anti-microbial coating on a medical device; and
   (b) bringing the silver material into contact with the alcohol or electrolyte to be treated so as to cause the release of the complex silver ions.

2. The method as set forth in claim 1, wherein the silver material forms one or more of the complex silver ions $Ag(CN)_2^-$, $AgCN_{(aq)}$ (ion pair), $Ag(NH_3)_2^+$, $AgCl_2^-$ and $Ag(S_2O_3)_2^{3-}$ in contact with an alcohol or a water based electrolyte.

3. , The method as set forth in claim 1 or 2, wherein the silver material is prepared as a powder, solution or suspension containing one or more of the complex silver ions.

4. The method as set forth in claim 1 or claim 3, wherein the silver material forms one or more of the complex silver ions $Ag(CN)_2^-$, $AgCN_{(aq)}$ (ion pair) and $Ag(NH_3)_2^+$ in contact with the alcohol or electrolyte.

## Patentansprüche

1. Verfahren zum Erzeugen einer antimikrobiellen Wirkung in einem Alkohol oder einem Elektrolyten auf Wasserbasis, umfassend:

   (a) Herstellen eines Silbermaterials derart, dass es andere Silber-Komplexionen als $Ag^+$, $Ag^{2+}$, $Ag^{3+}$, $Ag(OH)_2^-$, $Ag_2(OH)_3^-$ oder $Ag_3(OH)_4^-$ in einer Menge bildet, durch die in Kontakt mit einem Alkohol oder einem Elektrolyten auf Wasserbasis eine antimikrobielle Wirkung erzeugt wird, die größer ist, als diejenige, die durch eine entsprechende Menge an Silber in Form von $Ag^+$ erzeugt wird, wobei das Silbermaterial entweder feinkörnig oder als nanokristallines Pulver vorliegt, wahlweise zur Verwendung beim Herstellen einer antimikrobiellen Verbindung vor Ort oder in Form einer antimikrobiellen Beschichtung auf einem medizinischen Gerät, und
   (b) in-Kontakt-bringen des Silbermaterials mit dem Alkohol oder dem Elektrolyten, um es so zu behandeln, dass die Silber-Komplexionen freigesetzt werden.

2. Verfahren nach Anspruch 1,
   wobei das Silbermaterial in Kontakt mit einem Alkohol oder einem Elektrolyten auf Wasserbasis eine oder mehrere der Silber-Komplexionen $Ag(CN)_2^-$, $Ag(CN)_{(aq)}$(Ionenpaar), $Ag(NH_3)_2^+$, $AgCl_2^-$ und $Ag(S_2O_3)_2^{3-}$ bildet.

3. Verfahren nach einem der Ansprüche 1 oder 2,
   wobei das Silbermaterial als Pulver, Lösung oder Suspension hergestellt wird, das bzw. die eines oder mehrere der Silber-Komplexionen enthält.

4. Verfahren nach einem der Ansprüche 1 oder 3,
   wobei das Silbermaterial in Kontakt mit dem Alkohol oder dem Elektrolyten eines oder mehrere der Silber-Komplexionen $Ag(CN)_2^-$, $Ag(CN)_{(aq)}$(Ionenpaar) und $Ag(NH_3)_2^+$ bildet.

## Revendications

1. Procédé de production d'un effet anti-microbien dans un électrolyte à base d'alcool ou d'eau, comprenant les étapes qui consistent à :

   (a) préparer un matériau argenté tel qu'il forme des ions argent complexes autres que $Ag^+$, $Ag^{2+}$, $Ag^{3+}$, Ag

$(OH)_2^-$, $Ag_2(OH)_3^-$ ou $Ag_3(OH)_4^-$, en une quantité telle qu'elle permette de produire un effet anti-microbien au contact d'un alcool ou d'un électrolyte à base d'eau qui est supérieur à celui produit par une quantité équivalente d'argent sous la forme $Ag^+$, le matériau argenté étant sous la forme de grains fins ou de poudre nanocrystalline, optionnellement pour être utilisé dans la préparation d'une composition anti-microbienne topique, ou sous la forme d'un revêtement anti-microbien sur un dispositif médical ; et

(b) amener le matériau argenté au contact de l'alcool ou de l'électrolyte devant être traité de manière à entraîner la libération des ions argent complexes.

2.  Procédé selon la revendication 1, dans lequel le matériau argenté forme un ou plusieurs des ions argent complexes $Ag(CN)_2^-$, $AgCN_{(aq)}$ (paire d'ions), $Ag(NH_3)_2^+$, $Ag(Cl)_2^-$ et $Ag(S_2O_3)_2^{3-}$ au contact d'un alcool ou d'un électrolyte à base d'eau.

3.  Procédé selon la revendication 1 ou 2, dans lequel le matériau argenté est préparé sous la forme d'une poudre, d'une solution ou d'une suspension comprenant un ou plusieurs des ions argent complexes.

4.  Procédé selon la revendication 1 ou la revendication 3, dans lequel le matériau argenté forme un ou plusieurs des ions argent complexes $Ag(CN)_2^-$, $AgCN_{(aq)}$ (paire d'ions) et $Ag(NH_3)_2^+$ au contact de l'alcool ou de l'électrolyte.

Fig. 2.

Fig. 1.